# EUROPEAN PATENT APPLICATION

(11) **EP 2 036 598 A1**
(43) Date of publication of application: **18.03.2009**
(21) Application number: 07116599.7
(22) Date of filing: 17.09.2007
(51) Int. Cl.: A63F 13/10, A61B 5/00

(54) **Method and system for stimulating fitness**

(71) Applicant: Nederlandse Organisatie voor Toegepast- Natuurwetenschappelijk Onderzoek TNO, 2628 VK Delft (NL)
(72) Inventor: The designation of the inventor has not yet been filed
(74) Representative: Hatzmann, Martin

(57) **Abstract**

Method and system for stimulating the fitness of a user (1), comprising means (1,9) for playing an interactive computer game by the user, the game including a representation of the user, hereinafter called avatar (3), to which avatar one or more avatar parameters (PAR) can be assigned. A fitness instrument (4) is arranged to measure the user's fitness and to output fitness data (DAT) which represent the user's fitness. Data transfer means (5-8) are arranged for transferring the fitness data from the fitness instrument to the game. The computer game is enabled for setting or adjusting said avatar parameters of the user's avatar in accordance with the fitness data output by the fitness instrument. Moreover, the computer game is enabled for running the game, wherein the user may maintain or enhance the avatar's gaming behaviour by enhancing and measuring the user's fitness and transferring the updated fitness data to the game.

## Description

The present invention relates to a method for stimulating the fitness of a user.

Good physical fitness is a key to maintaining good health and fighting disease. Medical evidence has shown that excess body fat, for example, poses a major threat to health and longevity. Excess body fat is linked to major physical threats, such as heart disease, cancer, and diabetes. Consistent exercise, e.g. using fitness apparatus or fitness instruments etc. in various forms may be an important factor in maintaining proper weight and good health. Motivation plays a significant role in one's interest in continuous and productive exercise. Although exercise videos, programming, and literature abound, many people quickly lose interest in such short-term motivational tools.

US2005101845 shows a system comprising a (mobile) terminal and at least one sensor configured to sense a parameter associated with a user's physiology. One or more parameters associated with the user's physiology are sensed, the sensed one or more parameters defining physiological data associated with the user. The physiological data are analyzed relative to training regimen data, the training regimen data comprising one or both of range limits and durations associated with an exercise regimen. The physiological data are transferred to the terminal, and the terminal provides the physiological data in a form suitable for integration into an avatar representative of the user. The physiological data are processed in a physiological model and the results are illustrated by the form of the avatar displayed on the terminal screen. A disadvantage of the prior art system known from US2005101845 is that the output is rather static en does not include much incentive to the users to do fitness training.

One aim of the present invention is to stimulate condition or fitness training in any appropriate form, where any "fitness instrument" can be used to measure - and possibly to support - the condition or fitness of humans. Within the scope of this application, fitness instruments are deemed to include any kind of device which can be used for (at least) measuring data of the human body which may be related to the human's fitness or physical or physiological condition ("fitness data"), including step counters, heartbeat meters, blood pressure meters, meters for measuring weight, size of e.g. the human's belly, chest, muscles etc. Besides, some types of fitness instruments (like e.g. rowing and cycling machines etc.) may additionally be used for training the human body, in order to enhance the human's physical/physiological condition, which in turn will be reflected by more favourable fitness data (e.g. lower blood pressure, smaller belly size, larger muscles etc.).

According to the invention a method is preferred for stimulating the fitness of a user, which preferred method comprising steps of:
- providing an interactive computer game to be played by the user, the game including a representation of the user, hereinafter called avatar, to which avatar one or more avatar parameters can be assigned;
- providing a fitness instrument, arranged to measure the user's fitness and arranged to output fitness data which represent the user's fitness;
- providing data transfer means, arranged for transferring the fitness data from the fitness instrument to the game;
- setting or adjusting said avatar parameters of the user's avatar in accordance with the fitness data output by the fitness instrument;
- running the game, wherein the user may maintain or enhance the avatar's gaming behaviour by enhancing and measuring (again) the user's fitness and transferring the updated fitness data to the game.

In the way depicted here the user will be able to play a computer game, in which game (s)he is represented by an avatar. Within the game environment the avatar has to perform virtual physiological actions, like e.g. sprinting, jumping, reacting in dangerous situations etc., as is usual in (action) games. One or more avatar parameters, determining the avatar's performance or behaviour (e.g. speed, force, endurance) in the game, are assigned to the avatar. Besides, the avatar parameters may include parameters which influence the shape (e.g. thick & fat or lean & muscular) or the size of the avatar, which may also reflect the avatar's "power".

The parameters assigned to the user's avatar may initially be set on a default level or may, as may be preferred, be derived from an initial (real world) fitness test (sequence), to be done by the user on a fitness instrument, which outputs fitness data representing the user's physiological fitness (e.g. speed, force, endurance, weight, belly size etc.). When, during the game (e.g. against the computer or against other gamers via the Internet), the user notices that his/her avatar is not (or no more) doing very well in the game due to e.g. shortage of (reaction) speed, force, endurance, obesitas, overweight etc., this can be improved by training (in the real world) of the user himself/herself. In this way doing well (depending on the qualities of the avatar, determined by the avatar parameters) in a computer game is made dependent on the fitness level of the user, providing an incentive for the user to bring his/her condition to a higher level, viz. in order to win computer games.

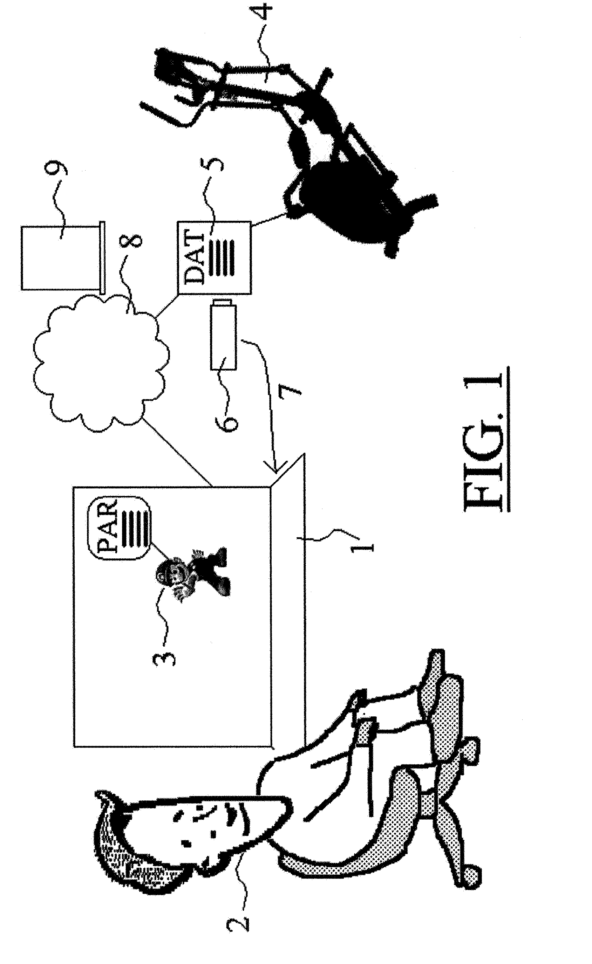

Figure 1 shows schematically an exemplary embodiment of a system according to the invention in which the foregoing method may be executed.

The system for stimulating the fitness of a user, shown in figure comprises means, represented by any type of gaming computer 1 (incl. TV, PlayStation™, PC etc.), for playing an interactive computer game by the user 2, the game including a representation of the user 2, called the user's avatar 3, to which avatar one or more parameters PAR can be assigned, ruling the avatar's gaming behaviour, performance and/or visualisation (shape, colour etc.). A fitness instrument 4 is arranged to enhance or at least to measure the user's physical and/or physiological fitness related characteristics and, moreover, arranged to output fitness data DAT, by means of a data recorder 5 which represent the user's fitness. So, the user 2 has to use the fitness instrument 4 at least to measure (e.g. by means of logging, recording) the user's fitness data. The user's fitness condition may, besides, be enhanced by either training on the fitness instrument or by training without the fitness instrument, e.g. by (Nordic) walking, swimming etc. In the latter case the fitness instrument can be used for only measuring the user's fitness data DAT. It is noted that, as an alternative, the user's fitness data DAT may be measured as well by other types of fitness instruments as shown in figure 1, e.g. portable, e.g. wristwatch like devices etc., but also his/her weight by a scale (the weight could be entered by means of a keyboard if the scale doesn't have a digital data output). Within the scope of this patent application such alternative fitness measuring devices will be considered to be included within the term "fitness instruments", the function of which might be restricted to measure the user's fitness or condition, but which, besides, additionally may be used to enhance the user's condition by exercising (e.g. rowing, cycling, boxing etc.)

The fitness data DAT thus may be recorded in the data recorder 5, which is, moreover, enabled to facilitate transfer of de fitness data DAT to the computer 1 and its gaming environment. The data transfer means, arranged for transferring the fitness data from the fitness instrument to the game, may be formed, as illustrated in figure 1, by the data recorder 5 and e.g. any form of a data carrier 6, like a USB stick, memory card etc., which can be written by the data recorder 5. The user 2 may take the data carrier 6 and connect it (7) to the computer 1, which, under control of the game software, can read the fitness data DAT. Another way is to transfer the data DAT via any (fixed or mobile) network 8 - e.g. via a wristwatch type fitness (measuring) instrument and a mobile telephone - to either the computer 1 or a game server 9. In either case de fitness data DAT may be converted, either in a game server 9 or within the local gaming environment within the computer 1, into corresponding avatar parameters PAR assigned to the user's avatar 3, e.g. by means of a physiological performance model, residing in either the server 9 or the computer 1, which model is arranged to convert the fitness data DAT into the avatar's 3 gaming parameters PAR which match with the user's actual physiological performance and/or other fitness related data.

Thus the computer game is enabled for setting or adjusting said gaming behaviour of the user's avatar 3, represented by the parameters PAR, in accordance with the fitness data DAT, output by the fitness instrument 4. The computer game is enabled to run the game, wherein the user 2 may maintain or enhance the avatar's 3 gaming behaviour by enhancing and measuring again the user's fitness and transferring the updated fitness data to the game, thus promoting - after all the user 2 will strive to win the computer game via the performance and behaviour of his/her avatar 3 - that the user 2 comes out of his/her lazy chair and starts training for the benefit of his/her own health and for the virtual health of his/her avatar 3, thus getting better health as well as doing well in the computer game.

## Claims

1. Method for stimulating the fitness of a user, comprising steps of:
- providing an interactive computer game to be played by the user, the game including a representation of the user, hereinafter called avatar, to which avatar one or more avatar parameters can be assigned;
- providing a fitness instrument, arranged to measure the user's fitness and arranged to output fitness data which represent the user's fitness;
- providing data transfer means, arranged for transferring the fitness data from the fitness instrument to the game;
- setting or adjusting said avatar parameters of the user's avatar in accordance with the fitness data output by the fitness instrument;
- running the game, wherein the user may maintain or enhance the avatar's gaming behaviour by enhancing and measuring the user's fitness and transferring the updated fitness data to the game.

2. Method according to claim 1, wherein the fitness data output by the fitness instrument, are converted into corresponding avatar parameters assigned to the user's avatar, by means of a physiological performance model, arranged to convert the fitness data into a behaviour of the avatar which matches with the user's actual physiological performance.

3. System for stimulating the fitness of a user (1), comprising:
- means (1,9) for playing an interactive computer game by the user, the game including a representation of the user, hereinafter called avatar (3), to which avatar one or more avatar parameters (PAR) can be assigned;
- a fitness instrument (4), arranged to measure the user's fitness and arranged to output fitness data (DAT) which represent the user's fitness;
data transfer means (5-8), arranged for transferring the fitness data from the fitness instrument to the game;
- the computer game being enabled for setting or adjusting said avatar parameters of the user's avatar in accordance with the fitness data output by the fitness instrument;
- the computer game being enabled for running the game, wherein the user may maintain or enhance the avatar's gaming behaviour by enhancing and measuring the user's fitness and transferring the updated fitness data to the game.

4. System according to claim 3, comprising processing means (1,9) arranged for converting the fitness data output by the fitness instrument into corresponding avatar parameters assigned to the user's avatar, by means of a physiological performance model, arranged to convert the fitness data into a behaviour of the avatar which matches with the user's actual physiological performance.

5. Software program product for carrying out the method according to claim 1 or 2.
